Europäisches Patentamt

European Patent Office

Office européen des brevets

(19)

(11) Publication number: **0 370 710**
**A2**

(12) # EUROPEAN PATENT APPLICATION

(21) Application number: **89311941.2**

(22) Date of filing: **17.11.89**

(51) Int. Cl.5: **C12N 15/40, C12Q 1/70**

(30) Priority: **19.11.88 GB 8827094**

(43) Date of publication of application:
**30.05.90 Bulletin 90/22**

(84) Designated Contracting States:
**AT BE CH DE ES FR GB GR IT LI NL SE**

(71) Applicant: **SCOTTISH CROP RESEARCH INSTITUTE**
**Invergowrie**
**Dundee DD2 5DA Scotland(GB)**

(72) Inventor: **Mayo, Michael Andrew**
**59 High Street Monifieth**
**Dundee DD5 4AA Scotland(GB)**
Inventor: **Reavy, Brian**
**16 Fountainbrae Monifieth**
**Dundee DD5 4DX Scotland(GB)**

(74) Representative: **Pattullo, Norman et al**
**Murgitroyd and Company Mitchell House 333 Bath Street**
**Glasgow G2 4ER(GB)**

(54) **DNA sequence encoding the coat protein gene of potato leafroll virus.**

(57) A DNA sequence characterised in that it is capable of hybridising with part of the RNA genome of at least one isolate of PLRV and is capable of transcription to yield RNA that encodes the coat protein of PLRV.

EP 0 370 710 A2

## DNA Sequence Encoding The Coat Protein Gene Of Potato Leafroll Virus

Potato leafroll virus (PLRV) is a virus that is spread from plant to plant by aphid vectors and that causes a serious disease of potato crops. It is a luteovirus, a group of viruses whose members are characterised by being transmitted in the persistent manner by vector aphids, and by having isometric particles which contain the viral genome of single-stranded RNA and which are largely confined to the phloem tissue of infected plants.

This invention provides a DNA sequence characterised in that it is capable of being transcribed to produce RNA which itself is capable of being translated to yield the coat (virus particle) protein of PLRV.

The invention is also substantially the following DNA sequence:

```
          10                   30                   50
ATAAATTCTTAGCGGGATTTGCTTTAGGATTCTCATCCGCAATCCCATTTTCAGTAGC
          70                   90                  110
CGGTTTATATTTTGTTTACCTAAAGATTTCCTCCCACGTGCGATCAATTGTTAATGAGTA
         130                  150                  170
CGGTCGTGGTTAAAGGAAATGTCAATGGTGGTGTACAACAACCAAGAATGCGAAGAAGGC
         190                  210                  230
```

AATCCCTTCGCAGGCGCGCTAACAGAGTTCAGCCAGTGGTTATGGTCACGGCCCCTGGGC
250　　　　　　　270　　　　　　　290

AACCCAGGCGCCGAAGACGCAGAAGAGGAGGCAATCGCCGCTCAAGAAGAACTGGAGTTC
310　　　　　　　330　　　　　　　350

CCCGAGGACGAGCTCAAGCGAGACATTCGTGTTTACAAAGGACAACCTCGTGGGCAACA
370　　　　　　　390　　　　　　　410

CCCAAGGAAGTTTCACCTTCGGGCCGAGTCTATCAGACTGTCCGGCATTCAAGGATGGAA
430　　　　　　　450　　　　　　　470

TACTCAAGGCCTACCATGAGTATAAGATCACAAGCATCTTACTTCAGTTCGTCAGCGAGG
490　　　　　　　510　　　　　　　530

CCTCTTCCACCTCCTCCGGTTCCATCGCTTATGAGTTGGACCCCCATTGCAAAGTATCAT
550　　　　　　　570　　　　　　　590

CCCTCCAGTCCTACGTCAACAAGTTCCAAATTACGAAGGCGGCGCCAAAACTTATCAAG
610　　　　　　　630　　　　　　　650

CGCGGATGATAAACGGGGTAGAATGGCACGATTCTTCTGAGGATCAGTGCCGGATACTGT
670　　　　　　　690　　　　　　　710

GGAAGGGAAATGGAAAATCTTCAGATTCCGCAGGATCCTTCAGAGTCACCATCAAGGTAG
730　　　　　　　750　　　　　　　770

CTTTGCAAAACCCCAAATAGGTAGACTCCGGATCAGAGCCTAGTCCAAGCCCACAACCAA
790　　　　　　　810　　　　　　　830

CACCCACTCCAACTCCCCAGAAGCACGAGCGATTTATTGCTTACGTTGGCATACCTATGC
850　　　　　　　870　　　　　　　890

TAACCATTCAGGCCAGGGAGAACGACGACCAAATCATATTGGGTTCCTTAGGGAGCCAAA
910　　　　　　　930　　　　　　　950

GGATGAAATATATAGAGGACGAGAACCAGAATTATACAAAGTTTAGTTCTGAGTATTACT
970　　　　　　　990

CTCAATCGAGCATGCAAGCCGTCCCTATGTATTACTTCAATG

This sequence, with its 23K coat protein translation product, is:

10　　　　　　　30　　　　　　　50
ATAAATTCTTAGCGGGATTTGCTTTAGGATTCTCATCCGCAATCCCATTTTCAGTAGC
70　　　　　　　90　　　　　　　110
CGGTTTATATTTTGTTTACCTAAAGATTTCCTCCCACGTGCGATCAATTGTTAATGAGTA
　　　　　　　　　　　　　　　　　　　　　M　S　T
130　　　　　　　150　　　　　　　170
CGGTCGTGGTTAAAGGAAATGTCAATGGTGGTGTACAACAACCAAGAATGCGAAGAAGGC
　V　V　V　K　G　N　V　N　G　G　V　Q　Q　P　R　M　R　R　Q
190　　　　　　　210　　　　　　　230
AATCCCTTCGCAGGCGCGCTAACAGAGTTCAGCCAGTGGTTATGGTCACGGCCCCTGGGC

3

```
     S  L  R  R  R  A  N  R  V  Q  P  V  V  M  V  T  A  P  G  _Q
          250                270                290
AACCCAGGCGCCGAAGACGCAGAAGAGGAGGCAATCGCCGCTCAAGAAGAACTGGAGTTC
     P  R  R  R  R  R  R  G  N  R  R  S  R  R  T  G  V  P
          310                330                350
CCCGAGGACGAGGCTCAAGCGAGACATTCGTGTTTACAAAGGACAACCTCGTGGGCAACA
     R  G  R  G  S  S  E  T  F  V  F  T  K  D  N  L  V  G  N  T
          370                390                410
CCCAAGGAAGTTTCACCTTCGGGCCGAGTCTATCAGACTGTCCGGCATTCAAGGATGGAA
     Q  G  S  F  T  F  G  P  S  L  S  D  C  P  A  F  K  D  G  I
          430                450                470
TACTCAAGGCCTACCATGAGTATAAGATCACAAGCATCTTACTTCAGTTCGTCAGCGAGG
     L  K  A  Y  H  E  Y  K  I  T  S  I  L  L  Q  F  V  S  E  A
          490                510                530
CCTCTTCCACCTCCTCCGGTTCCATCGCTTATGAGTTGGACCCCCATTGCAAAGTATCAT
     S  S  T  S  S  G  S  I  A  Y  E  L  D  P  H  C  K  V  S  S
          550                570                590
CCCTCCAGTCCTACGTCAACAAGTTCCAAATTACGAAGGGCGGCGCCAAAACTTATCAAG
     L  Q  S  Y  V  N  K  F  Q  I  T  K  G  G  A  K  T  Y  Q  A
          610                630                650
CGCGGATGATAAACGGGGTAGAATGGCACGATTCTTCTGAGGATCAGTGCCGGATACTGT
     R  M  I  N  G  V  E  W  H  D  S  S  E  D  Q  C  R  I  L  W
          670                690                710
GGAAGGGAAATGGAAAATCTTCAGATTCCGCAGGATCCTTCAGAGTCACCATCAAGGTAG
     K  G  N  G  K  S  S  D  S  A  G  S  F  R  V  T  I  K  V  A
          730                750                770
CTTTGCAAAACCCCAAATAGGTAGACTCCGGATCAGAGCCTAGTCCAAGCCCACAACCAA
     L  Q  N  P  K
          790                810                830
CACCCACTCCAACTCCCCAGAAGCACGAGCGATTTATTGCTTACGTTGGCATACCTATGC
          850                870                890
TAACCATTCAGGCCAGGGAGAACGACGACCAAATCATATTGGGTTCCTTAGGGAGCCAAA
          910                930                950
GGATGAAATATATAGAGGACGAGAACCAGAATTATACAAAGTTTAGTTCTGAGTATTACT
          970                990
CTCAATCGAGCATGCAAGCCGTCCCTATGTATTACTTCAATG
```

In a further aspect the invention provides a bacterial plasmid characterised in that it contains a DNA sequence capable of hybridising with a portion of the RNA genome of PLRV.

The bacterial plasmid has the DNA sequence containing the coding sequence specifying the coat protein of PLRV which when brought into contact with suitable plant cells will cause some cells to be transformed with the plasmid sequences containing the PLRV-specific DNA.

The invention also provides a method of producing a duplex DNA sequence capable of hybridising with a portion of the genome RNA of at least one isolate of PLRV which comprises:

    a) isolating RNA from purified PLRV particles;

    b) synthesising DNA copies of a portion of the RNA by bringing said RNA into contact with a reverse transcriptase in the presence of a synthetic oligonucleotide primer under conditions of time and temperature sufficient for the production of complementary DNA (cDNA); and

    c) synthesising duplex DNA by bringing cDNA-RNA hybrids into contact with ribonuclease H and a DNA polymerase under conditions of time and temperature sufficient for the production of duplex DNA.

In yet another aspect the invention provides a DNA probe useful for the detection of PLRV and characterised as comprising a DNA sequence complementary to a portion of the RNA genome of PLRV and capable of hybridisation therewith.

In yet another aspect the invention provides a method of detection of PLRV in a sample containing PLRV and which comprises:

    a) nick-translation or oligo-labelling of plasmid DNA containing the PLRV-specific DNA sequence in the presence of radioactive or otherwise labelled deoxyribonucleoside triphosphate;

b) applying samples of suspected infected plants or of suspected viruliferous insects to a support, for example a sheet of nitrocellulose or other membrane, to bind the RNA components;

c) treating the support with the labelled DNA under conditions such that the DNA can hybridise to complementary or substantially complementary RNA sequences; and

d) washing the support under conditions such that essentially only hybridised labelled DNA is retained and detecting this bound DNA.

In another aspect the invention provides a method of producing RNA that is capable of hybridising with a portion of the genome RNA of at least one isolate of PLRV and that comprises:

a) isolating the PLRV specific DNA sequence from the plasmid cloning vector;

b) inserting the PLRV specific DNA sequence into a transcription vector;

c) copying the DNA sequence to produce labelled RNA for use in detecting PLRV RNA as in the above-defined aspect of the invention;

d) applying samples of suspected infected plants or of suspected viruliferous insects to a support, for example a sheet of nitrocellulose or other membrane, to bind the RNA components;

e) treating the support with the labelled RNA under conditions such that the RNA can hybridise to complementary or substantially complementary RNA sequences; and

f) washing the support under conditions such that essentially only hybridised labelled RNA is retained and detecting this bound RNA.

In a further aspect the invention provides a method of producing PLRV coat protein comprising culturing a host cell under conditions promoting the synthesis of PLRV coat protein and in which said host cell is further characterised as being transformed by an expression vector comprising the DNA sequence in an orientation with a promoter sequence such that in the host the said DNA is expressed to produce PLRV coat protein.

In a further aspect the invention provides a method of transforming plants such that they express the DNA sequence to produce PLRV coat protein either transiently or constitutively and exemplified by:

a) excising at least a portion of the PLRV-specific DNA sequence such that the excised portion comprises some or all of the PLRV coat protein-coding region;

b) inserting the excised portion into an expression cassette of a plasmid;

c) transforming plant cells or protoplasts, with a plasmid including the expression cassette;

d) selecting the transformed cells and regenerating transformed plants from them.

The plasmid expression cassette may include elements of Agrobacterium tumefasciens Ti plasmid. The plant cells or protoplasts are for example those of potato plants.

The invention can provide a PLRV-specific sequence for transforming plant cells by any method such that the transformed cells express the DNA sequence to produce PLRV coat protein or part thereof.

This invention relates to the cloning of genetic information of potato leafroll virus. Such an invention is useful as a probe for the detection of PLRV genomes in samples, for the production of PLRV coat protein by means of recombinant DNA techniques and for the production of transgenic cells and plants that constitutively synthesise PLRV coat protein.

The term "PLRV" in the foregoing part of this specification and in the appended Claims is used to denote potato leafroll virus and related viruses which may take the form of different strains of PLRV or closely similar but different viruses, such for example as tomato yellow top virus.

The terms "coat protein" and/or "virus particle protein" in this specification are used to relate to the 23k coat protein alone and/or fusion of the 23k coat protein with all or part of the product of the 53k protein gene encoded after the 3' end of the 23k coat protein gene and characterised in that the fusion proteins contain the 23k coat protein at their N-terminal ends and are produced by translational readthrough of the 23k coat protein termination codon.

Embodiments of the present invention will now be described by way of illustration only, in the following Examples, with reference to the accompanying diagrams in which:

Fig. 1 is a diagram showing the positions of

(a) relevant restriction enzyme sites,

(b) the coat protein coding region,

(c) the sequence of a selective probe and

(d) the sequence contained in pPLR439;

Fig. 2 is a diagram showing the construction of a plasmid of the invention designed to test the expression of the coat protein gene in animal cells;

Fig. 3 is a diagram illustrating the construction of a plasmid of the invention used for transformation of plants;

Fig. 4 is the sequence of the 23K coat protein and 53K readthrough region and the gene sequence;

Fig. 5 is a chart of the in vitro translation products of PLRV-specific RNA transcripts;

Fig. 6 is a chart of the Northern blots of RNA extracted from normal and transgenic plants; and

Fig. 7 is the DNA sequence of the invention with the 23K coat protein.

EXAMPLE 1

The rationale employed for the cloning of genetic information of PLRV was as follows: PLRV was propagated by growing potato plants from PLRV-infected potato tubers and the virus particles purified by treating extracts of frozen leaf and stem tissue from these plants as described by Tamada & Harrison (1980) except that 5% Celluclast (Novo Enzyme Products Limited) was used in place of Driselase to macerate the vascular tissue. After this treatment the extract was clarified by solvent treatment and PLRV particles were recovered from the suspension by precipitation with polyethylene glycol and differential centrifugation as described by Tamada & Harrison (1980). PLRV particles were further purified by sedimentation in sucrose density gradients as described by Tamada & Harrison (1980). RNA was extracted from the purified particles as described by Mayo et al (1982) and recovered by precipitation from 70% ethanol. RNA was hybridised to oligonucleotide primers at 65 degrees centigrade in 50 mM tris-HCl, pH 7.5, 75 mM KCl, 3 mM magnesium chloride and 10 mM dithiothreitol for 2 minutes and cooled slowly for 90 minutes to 42 degrees centigrade.

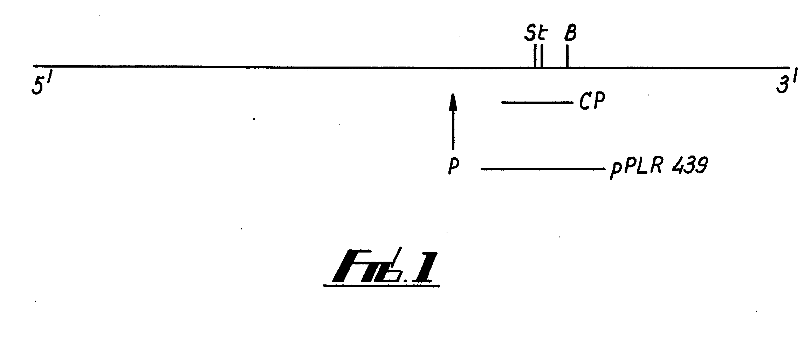

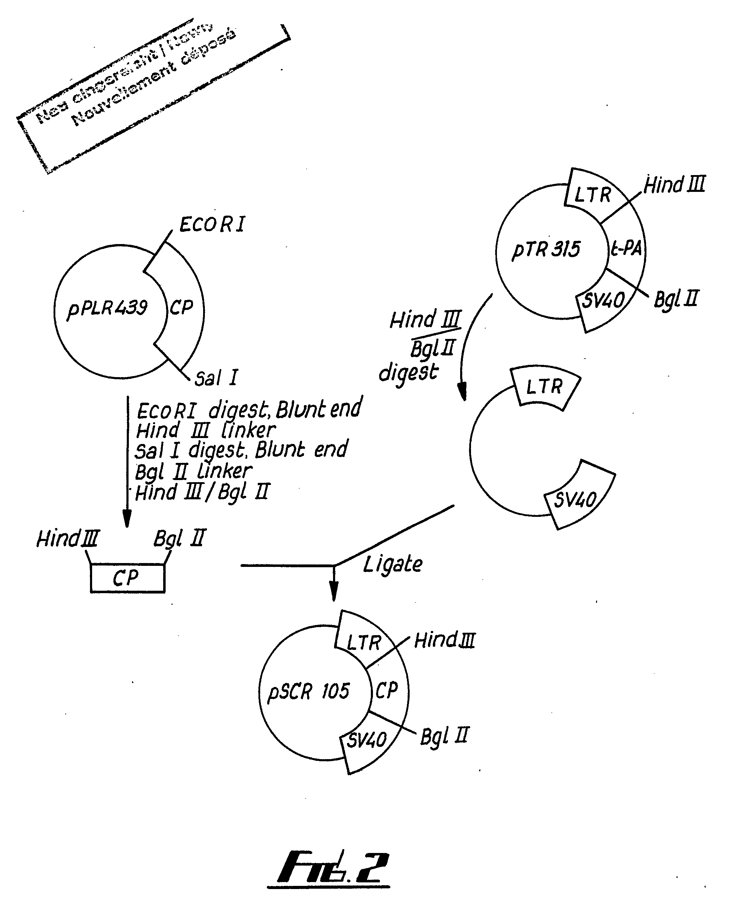

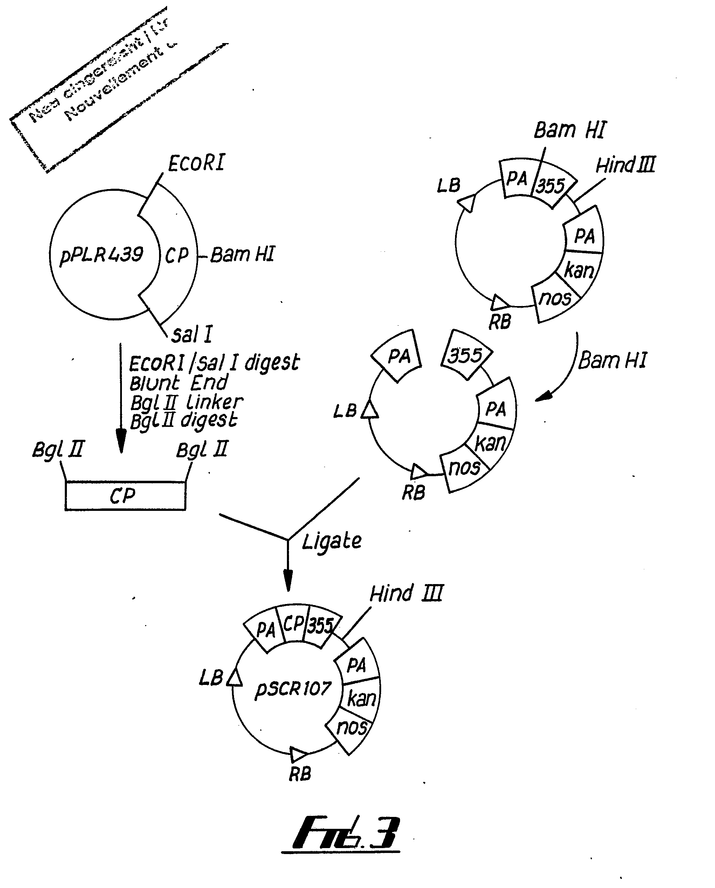

Double-stranded cDNA was made from the primed RNA using a cDNA Synthesis Kit (Amersham International) and then ligated into pUC 19 (Pharmacia), that had previously been linearised using restriction endonuclease SmaI, using T4 DNA ligase under conditions recommended by the manufacturer (BRL). Competent cells of Escherichia coli, strain DH5 alpha (BRL) were transformed with the recombinant plasmids under the conditions recommended by the manufacturer and suitable colonies of transformants were selected as lacking active B-galactosidase and being resistant to ampicillin and then by colony hybridisation (Maniatis et al 1982) using as a probe cDNA made to PLRV RNA as described by Taylor et al (1976). The sizes of the DNA inserts in the pUC 19 growing in these positively hybridising bacterial clones were determined by making small-scale preparations of plasmids, cutting the DNA with restriction endonucleases Sac I and Pst I and determining the size(s) of the DNA fragment(s) after subtracting the size attributable to the linearised pUC 19. Suitable clones were selected as those that contained two StuI sites, separated by 51 nucleotides, a BamHI site 217 nucleotides to the 3' side of the 3'-most of the Stu I sites, and which had at least 600 nucleotides to the 3' side of the BamHI site and at least 50 nucleotides to the 3' side of the BamHI site. The clone selected (pPLR439) had an insert size of about 1 kb and thus encompassed the coat protein coding region (Fig. 1). Its location was verified by determining the nucleotide sequence of restriction enzyme fragments of the DNA insert by dideoxy chain termination (Sanger et al 1977). The inclusion of the coat protein coding region within the sequence obtained was confirmed by establishing the existence of an open reading frame capable of being translated to give a polypeptide with the size of the coat protein of PLRV and by expressing the coding potential of the sequence in HeLa cells. In other experiments suitable clones were also selected at the colony blotting stage by using a 200-nucleotideprobe of single-stranded DNA with a sequence complementary to that ending about 200 nucleotides 5' of the coat protein initiation codon (see Fig. 1) that had been cloned in M13 bacteriophage and then P32 labelled as described by Barker et al (1985).

In Fig. 1, the symbols represent the following:

5' - the 5' end of PLRV RNA

3' - the 3' end of PLRV RNA

St - the 2 Stu I sites in pPLR 439

B - the BamH I site in pPLR 439

CP - the region coding for PLRV coat protein

P(arrowed) - the position of the selective probe

pPLR 439 - the position of the PLRV specific sequence in pPLR 439

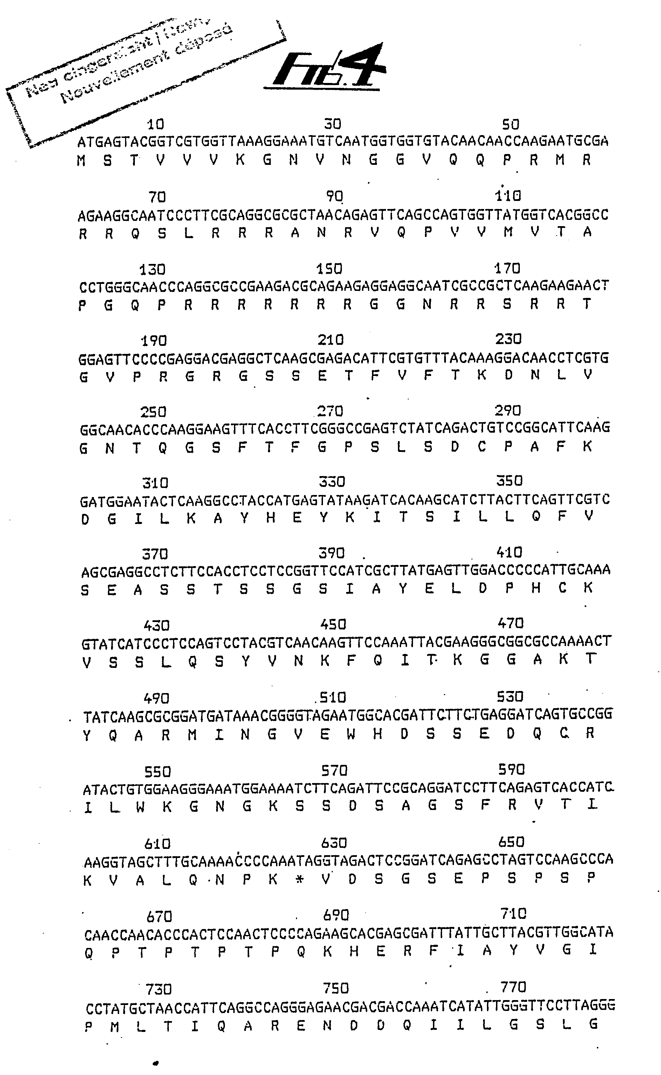

The vector for expression in HeLa cells was prepared as follows (see also Fig. 2).

In Fig. 2 the abbreviations represent the following:

CP - potato leafroll virus cDNA encoding the coat protein

LTR - rous sarcoma virus long terminal repeat

SV 40 - simian virus 40 small t antigen intron and polyadenylation signals

t-PA - human tissue-type plasminogen activator cDNA

EcoRI

Sal I

Hind III   } represent the positions of restriction endonuclease cleavage sites

Bgl II

Plasmid pPLR439 was cleaved to completion with EcoR I at the 5' end of the coat protein coding region. The DNA was purified by phenol/chloroform extraction and ethanol precipitation. The cohesive ends of the DNA were filled in using Klenow fragment of DNA polymerase I and Hind III linker DNA (Pharmacia) was ligated onto the blunt-ended DNA as described by Maniatis et al (1982). The DNA was then repurified as above and cleaved to completion with Sal I at the 3' end of the coat protein coding region. The DNA was then repurified, made to have blunt ends and ligated to Bgl II linker DNA as above. Following one or more repurifications, the DNA was cleaved with Hind III and Bgl II to generate cohesive ends for cloning into pTR 315 (Browne et al 1988) that had been cleaved with Hind III and Bgl II. Cleaved DNA was subjected to electrophoresis in 1% low melting point agarose (Serva), the appropriately sized fragments (ca. 1 kb for the coat protein coding region cDNA and ca. 5.5 kb for the pTR 315 fragment) were excised from the gel, purified and then ligated in a total volume of 10 ul as described by Maniatis et al (1982). 1 ul of the ligation reaction mixture was used to transform competent cells of E. coli strain HB 101 (BRL) as described by the manufacturers. Transformed cells of HB 101 were selected by their growth in the presence of ampicillin and the structure of the plasmid they contained was established by digesting plasmid prepared from cultures of the transformants by the alkaline lysis method (Birnboim & Doly 1979) with a mixture of Hind III and Bgl II. Suitable transformants contained plasmid containing a 1 kb insert fragment in the cloning site of pTR 315. This plasmid was named pSCR 105 and was transfected into HeLa cells by calcium phosphate co-precipitation followed by glycerol shock and sodium butyrate treatment as described by Gorman (1985). Transfected cells were then cultured for 24 hours at 37 degrees centigrade. The cells were then extracted by treatment with sample buffer and immunoblotted to nitrocellulose as described by Towbin et al (1979). The nitrocellulose sheets were reacted with a 1/100 dilution of monoclonal antibody SCR 1 which is specific for PLRV coat protein. Bound antibody was detected by reaction with alkaline phosphatase-conjugated anti-mouse antibodies followed by reaction of the bound enzyme to yield a coloured reaction product as recommended by the manufacturers (BioRad). The specificity of the reaction was established by similarly treating extracts of cells transfected with plasmid lacking the PLRV-specific DNA sequence.

The expression of the coding region to the 3' side of the coat protein gene was examined by in in vitro translation of RNA transcripts of the PLRV-specific DNA sequence in pPLR439. DNA was excised from pPLR439 by digestion with EcoRI and SalI, and ligated into EcoRI/SalI cut pBluescript (Stratagene) to produce pSCR103. RNA transcripts were produced essentially as described by Stratagene using SalI-linearised pSCR103 and T7 RNA polymerase.

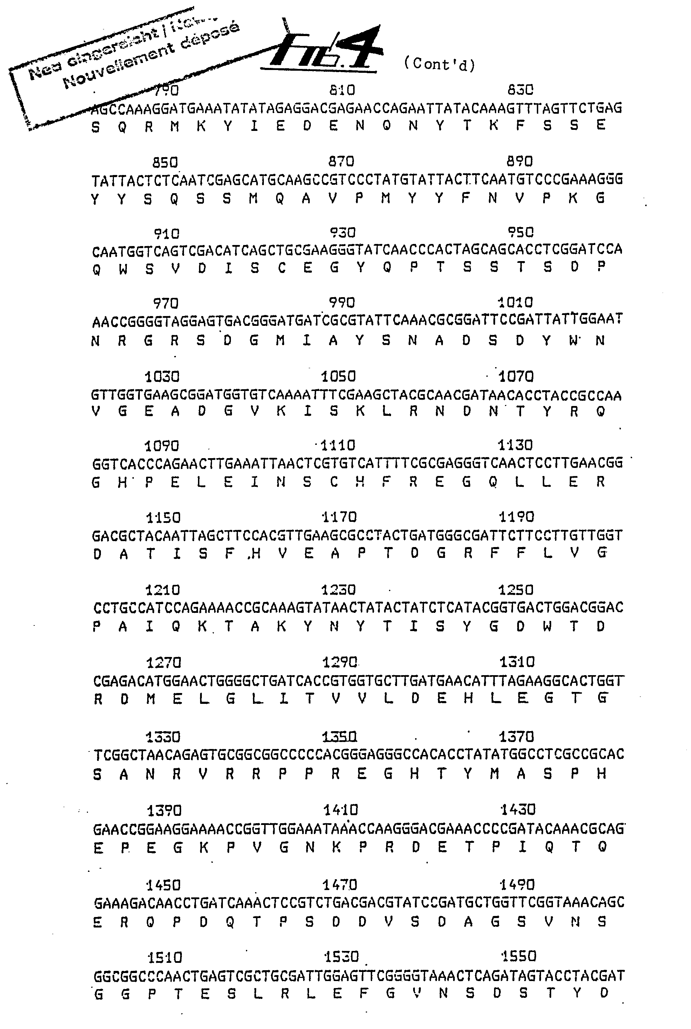

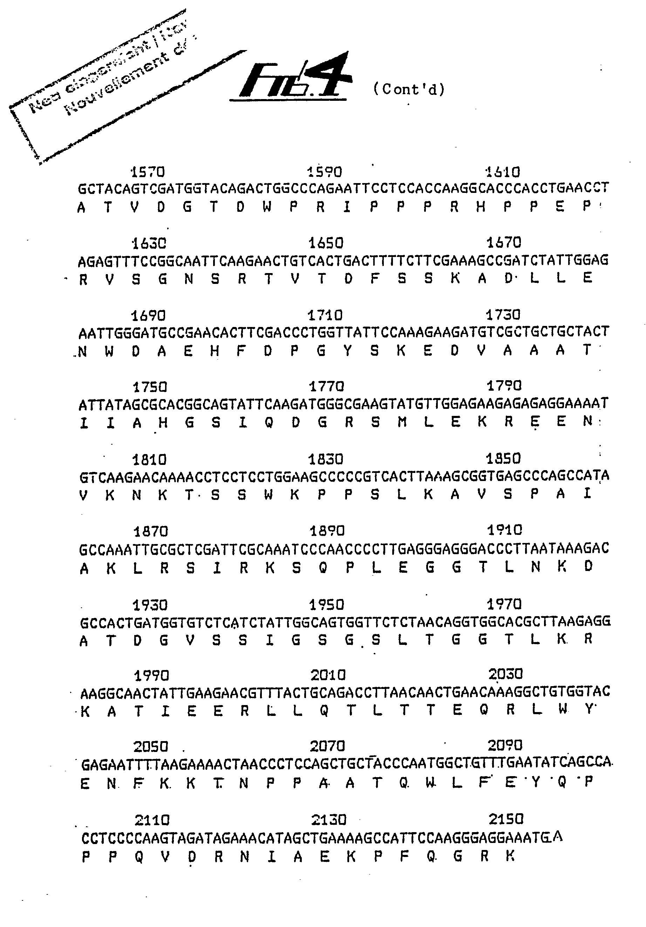

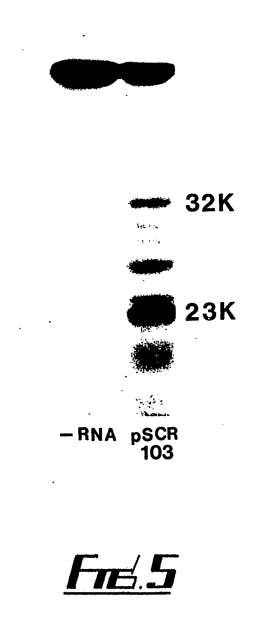

RNA transcripts were added to reticulocyte lysate translation mixtures (Amersham International) together with $^{35}$S-methionine. After 90 minutes at 30 degrees celsius the mixture was analysed by fluorgraphy. The radioactive polypeptides specific to transcript RNA include coat protein (Mr 23000, 23k) and a 32k polypeptide which is the size expected for translation of the entire transcript RNA including the termination codon for the 23k coat protein and for about 9k of the readthrough protein (Fig. 5). The complete sequence of the 23K coat protein and 53K readthrough region, and the sequence encoding it of (Mayo et al., 1989) are shown in Fig. 3.

Construction of the vector for expressing PLRV coat protein in plants was as follows (see also Fig. 3).

In Fig. 3 the abbreviations represent the following:

CP - potato leafroll virus cDNA encoding the coat protein

35S - cauliflower mosaic virus 35S promoter

LB - left t-DNA border of pTi T37

RB - right t-DNA border of pTi T37

Kan - Tn5 neomycin phosphotransferase

nos - nopaline synthase promoter

pA - nopaline synthase polyadenylation sequences

```
EcoRI     ⎫
Sal I     ⎬   represent the positions of restriction
Hind III  ⎨   endonuclease cleavage sites
Bgl II    ⎭
```

The sequence encoding the coat protein was excised from pPLR 439 by digestion with EcoR I and Sal I. This DNA fragment was made to have blunt ends and then ligated to Bgl II linker DNA as described above. Following digestion with Bgl II the fragment was cloned into the BamH I site of pROK 2, a Bin 19 (Bevan 1984) based vector containing a cauliflower mosaic virus 35S promoter and nopaline synthase polyadenylation sequences, as described above except that recombinant selection was made using resistance to kanamycin instead of ampicillin. Clones containing the PLRV coat protein coding region in the appropriate orientation were identified by analysing the sizes of the products of digestion by Hind III and BamHI of a plasmid preparation made by alkaline lysis as described above. Clones containing the insert in the correct orientation produce a fragment of about 1.5 kb and one such clone was named pSCR107. pSCR107 was mobilised from E. coli HB 101 into Agrobacterium LBA4404 by tri-parental mating using plasmid pRK2013 as described by (Armitage et al 1988). The presence of the plasmid in transformed agrobacteria was confirmed by an agrobacterium quick screen as described by An et al (1988) followed by appropriate restriction enzyme digestions.

This transformed agrobacterium (LBA107) was used to transform potato and Nicotiana clevelandii by conventional coculture methods (Draper et al., 1988). Transformed tissue was obtained by selecting kanamycin-resistant regenerants. These were then cultured to yield whole plants. The presence of PLRV-specific sequence in transformed material was tested by probing Northern blots of RNA extracts. The probe was oligo-labelled PLRV-specific DNA excised from pPLR439 by digestion with EcoRI and SalI.

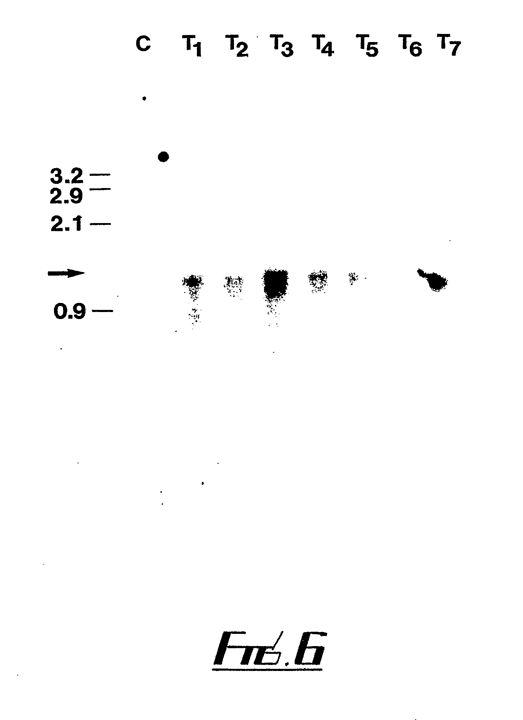

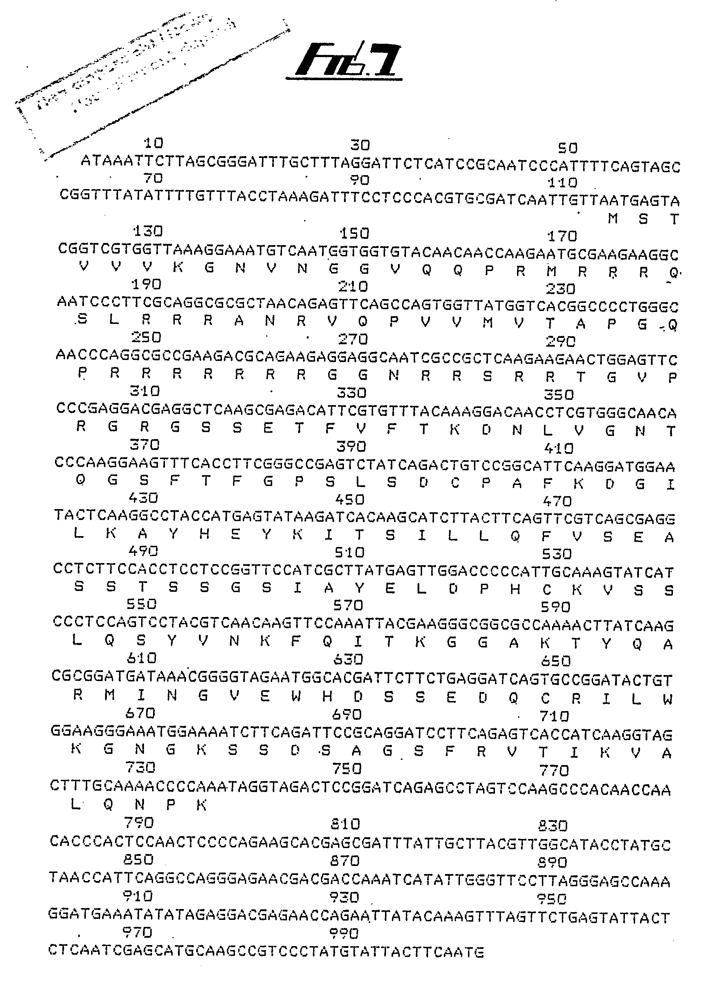

A 1kb PLRV-specific RNA species was detected in potato and N. clevelandii tissues indicating that the plant genomes contained PLRV-specific sequence and that this was transcribed by the plant cells (Fig. 6).

Potato plants transformed with the PLRV-specific sequence were more resistant to infection with aphid-transmitted PLRV than were control plants, and the virus multiplied to a smaller extent in those plants that became infected than it did in the control plants.

In Fig. 5 the track labelled "pSCR103" shows the PLRV-specific protein products: the 23k coat protein and 32k readthrough proteins are indicated on the right-hand side. The track labelled "-RNA" shows the endogenous products produced by the reticulocyte lysate.

In Fig. 6, track C is RNA from a normal, nontransformed plant; tracks T1-T6 are RNAs from transformed potato plants; track T7 is RNA from a transformed Nicotiana clevelandii plant. The positions and sizes (kb) of brome mosaic virus markers are indicated on the left. The arrow indicates the 1kb PLRV-specific RNA transcript detected using an oligo-labelled PLRV-specific DNA excised from pPLR439 by digestion with EcoRI and SalI.

## EXAMPLE 2

### Virus propagation and purification.

The PLRV isolate used was derived from strain 1, a field isolate (Tamada et al., 1984) obtained from infected tubers of the potato variety Cara grown in Scotland. The isolate was maintained in potato tubers and virus particles were extracted from infected potato shoots and leaves and purified as described by Tamada & Harrison (1980) except that 5% Celluclast (Novo Enzyme Products) was used in place of Driselase to macerate the vascular tissues.

### Extraction and analysis of RNA.

Virus particles were sedimented from sucrose fractions by high-speed centrifugation and RNA was extracted from the pellets as described by Mayo et al. (1982). The Mr of the RNA was estimated by electrophoresis of glyoxal-treated RNA in agarose gels. Mr markers were RNA from particles of brome mosaic and tobacco mosaic viruses, and Escherichia coli ribosomal RNA.

Molecular cloning

In some experiments RNA was polyadenylated in reaction mixtures containing 50 mM-TrisHClpH 7.9,0.25 M-NaCl,10mM-MgCl , 40ug bovine serum albumin, 0.8 mM-ATP, 5ug PLRV RNA and 2.5 units poly(A) polymerase in 100ul for 15 min at 37 degrees celsius, recovered and annealed to oligo(dT) as described by Maniatis et al. (1982). In other experiments RNA was annealed to synthetic oligonucleotide primers (Applied Biosystems). These were 5'TACTTCTTTCACGGAGTT3'(A)- ,5'CGCTTTCTTTTGTTTGGGC3' and 5'GGTTCTCGTCCTCTATATA 3'(B) which are complementary to nucleotides 641 to 658, 1954 to 1973 and 4488 to 4506, respectively, in the sequence of the PLRV RNA (Mayo et al 1989). Double-stranded DNA was synthesized by reverse transcription followed by second-strand DNA synthesis and S1 nuclease treatment (Maniatis et al., 1982) for the first cloning or, in some experiments, using a commercial kit (Amersham). Recombinant plasmids were made either by the addition of 3' oligo(dC) to the cDNA followed by annealing with PstI-cut and oligo(dG)-tailed pBR322 or pUC9, by the addition of EcoR1 linkers and ligation into EcoRI-cut pUC19, or by blunt-end ligation into SmaI-cut pUC19. The procedures were essentially those described by Maniatis et al. (1982) or as recommended by the suppliers of the enzymes used. Recombinant pBR322 was used to transform E. coli strain DH 1 made competent by treatment with CaCl₂, RbCl and MnCl₂ and strains JM101 and DH5 alpha (Bethesda Research Laboratories) were hosts for recombinant pUC plasmids, Candidate clones (ampicillin-sensitive pBR322 or lac-negative pUC) were tested by colony blotting (Maniatis et al., 1982) either with 32P-labelled cDNA to PLRV RNA made by random priming (Taylor et al., 1976) or with PLRV-specific sequences isolated from recombinant M13 bacteriophage DNA labelled as described by Barker et al. (1985).

Subcloning and sequencing.

Plasmids were prepared by alkaline lysis (Birnboim & Doly, 1979; Maniatis et al., 1982) and analysed for restriction sites by digestion and electrophoresis in polyacrylamide gels. Appropriate fragments of insert DNA were recovered by electroelution and spermine precipitation. DNA fragments were ligated into M13mp10 or M13mp11 replicative form (RF) DNA and sequenced by dideoxyribonucleotide chain termination (Sanger et al., 1977) and electrophoresis in buffer gradient 6% gels. Deoxy-7-deazaGTP was used in place of dGTP when Klenow polymerase was used. In some experiments modified T4 polymerase (Sequenase; Cambridge Bioscience) was used. Sequences were assembled using the progams DBUTIL and DBAUTO and analysed by ANALYSEQ, DIAGON or GAP.

In vitro translation.

RNA at 0.1 mg/ml was translated in a wheatgerm extract. Polypeptides were labelled with [35 S]-methionine and analysed by electrophoresis in 10% polyacrylamide gels and fluorography. Marker proteins were beta-galactosidase (Mr 116K, alpha-phosphorylase (97.4K), bovine serum albumin (66K), ovalbumin (45K) and carbonic anhydrase (29K) (Sigma).

A deposit of the bacterial culture of E Coli with the DNA sequence of the invention has been made at the National Collection of Industrial and Marine Bacteria Ltd (NCIMB), Torry Research Station, 135 Abbey Road, Aberdeen, Scotland on 2nd November 1988; the deposit number NCIB 40083 was assigned.

REFERENCES

AN G, EBERT P R, MITRA A, and HA S B (1988). Binary vectors. In 'Plant Molecular Biology Manual' A3, 1-19, Kluwer Academic Publishers, Dordrecht, Netherlands.

ARMITAGE, P, WALDEN, R & DRAPER, J (1988). In 'Plant Genetic Transformation and Gene Expression'. Edited by J Draper, R Scott, P Armitage & R Walden, pp1-67, Blackwell Scientific Publications, Oxford.

BARKER J M, McINNES J L, MURPHY P J & SYMONS R H (1985). Dot-blot procedure with (32P)-DNA probed for the sensitive detection of avocado sunblotch and other viroids in plants. Journal of Virological Methods 10, 87-98.

BIRNBOIM H C & DOLY J (1979). A rapid alkaline extraction procedure for screening recombinant plasmid DNA. Nucleic Acids Research 7, 1513-1523.

BEVAN M (1984). Binary agrobacterium vector for plant transformation. Nucleic Acids Research 12, 8711-8721.

BROWNE M J, CAREY J E, CHAPMAN C G, TYRREL A W R, ENTWHISTLE C, LAWRENCE G M P, REAVY B, DODD I, ESMAIL A & ROBINSON J H (1988). A tissue-type plasminogen activator mutant with prolonged clearance in vivo. The Journal of Biological Chemistry 263, 1599-1602.

DRAPER J, SCOTT R & HAMIL J (1988). In 'Plant Genetic Transformation and Gene Expression'. Edited by J Draper, R Scott, P Armitage & R Walden, pp71-160, Blackwell Scientific Publications, Oxford.

GORMAN C (1985). In 'DNA Cloning II' Edited by D M Glover, pp143-190, IRL Press Ltd, Oxford.

HARRISON, B D (1984). Potato leafroll virus. Commonwealth Mycological Institute/Association of Applied Biologists Descriptions of Plant Viruses, No 291.

MANIATIS T, FRITSCH E F & SAMBROOK J (1982). Molecular cloning: a laboratory manual. New York: Cold Spring Harbor Laboratory.

MAYO M A, BARKER H, ROBINSON D J, TAMADA T & HARRISON B D (1982). Evidence that potato leafroll virus is positive-stranded, is linked to a small protein and does not contain polyadenylate. Journal of General Virology 59, 163-167.

MAYO M A, ROBINSON D J, JOLLY C A, & HYMAN L (1989). Nucleotide sequence of potato leafroll luteovirus RNA. Journal of General Virology, 70 1037-1051.

SANGER F, NICKLEN S & COULSON A R (1977). DNA sequencing with chain terminating inhibitors. Proceedings of the National Academy of Sciences, USA 74 5463-5467.

TAMADA T & HARRISON B D (1980). Factors affecting the detection of potato leafroll virus in potato leafroll virus in potato foliage by enzyme-linked immunosorbent assay. Annals of Applied Biology 95, 209-219.

TAMADA T, HARRISON B D & ROBERTS I M (1984). Variation among British isolates of potato leafroll virus. Annals of Applied Biology 104, 107-116.

TAYLOR J M, ILLMENSEE R & SUMMERS J (1976). Efficient transcription of RNA into DNA by avian sarcoma virus polymerase. Biochimica et Biophysica Acta 442, 324-330.

TOWBIN H, STAEHELIN T & GORDON J (1979). Electrophoretic transfer of proteins from polyacrylamide gels to nitrocellulose sheets; procedure and some applications. Proceedings of the National Academy of Sciences, USA 76 4350-4354.

Modifications and improvements can be made without departing from the scope of the invention.

## Claims

1. A DNA sequence characterised in that it is capable of hybridising with part of the RNA genome of at least one isolate of PLRV and is capable of transcription to yield RNA that encodes the coat protein of PLRV.

2. A DNA sequence as claimed in Claim 1, wherein the sequence includes or consists of substantially the following:

```
             10                   30                   50
ATAAATTCTTAGCGGGATTTGCTTTAGGATTCTCATCCGCAATCCCATTTTCAGTAGC
             70                   90                  110
CGGTTTATATTTTGTTTACCTAAAGATTTCCTCCCACGTGCGATCAATTGTTAATGAGTA
            130                  150                  170
CGGTCGTGGTTAAAGGAAATGTCAATGGTGGTGTACAACAACCAAGAATGCGAAGAAGGC
            190                  210                  230
AATCCCTTCGCAGGCGCGCTAACAGAGTTCAGCCAGTGGTTATGGTCACGGCCCCTGGGC
            250                  270                  290
AACCCAGGCGCCGAAGACGCAGAAGAGGAGGCAATCGCCGCTCAAGAAGAACTGGAGTTC
            310                  330                  350
CCCGAGGACGAGCTCAAGCGAGACATTCGTGTTTACAAAGGACAACCTCGTGGGCAACA
            370                  390                  410
CCCAAGGAAGTTTCACCTTCGGGCCGAGTCTATCAGACTGTCCGGCATTCAAGGATGGAA
            430                  450                  470
TACTCAAGGCCTACCATGAGTATAAGATCACAAGCATCTTACTTCAGTTCGTCAGCGAGG
            490                  510                  530
CCTCTTCCACCTCCTCCGGTTCCATCGCTTATGAGTTGGACCCCCATTGCAAAGTATCAT
            550                  570                  590
CCCTCCAGTCCTACGTCAACAAGTTCCAAATTACGAAGGGCGGCGCCAAAACTTATCAAG
            610                  630                  650
CGCGGATGATAAACGGGGTAGAATGGCACGATTCTTCTGAGGATCAGTGCCGGATACTGT
            670                  690                  710
```

11

GGAAGGGAAATGGAAAATCTTCAGATTCCGCAGGATCCTTCAGAGTCACCATCAAGGTAG
730    750    770

CTTTGCAAAACCCCAAATAGGTAGACTCCGGATCAGAGCCTAGTCCAAGCCCACAACCAA
790    810    830

CACCCACTCCAACTCCCCAGAAGCACGAGCGATTTATTGCTTACGTTGGCATACCTATGC
850    870    890

TAACCATTCAGGCCAGGGAGAACGACGACCAAATCATATTGGGTTCCTTAGGGAGCCAAA
910    930    950

GGATGAAATATATAGAGGACGAGAACCAGAATTATACAAAGTTTAGTTCTGAGTATTACT
970    990

CTCAATCGAGCATGCAAGCCGTCCCTATGTATTACTTCAATG
   10    30    50
ATAAATTCTTAGCGGGATTTGCTTTAGGATTCTCATCCGCAATCCCATTTTCAGTAGC
   70    90    110
CGGTTTATATTTTGTTTACCTAAAGATTTCCTCCCACGTGCGATCAATTGTTAATGAGTA
                 M S T
   130    150    170
CGGTCGTGGTTAAAGGAAATGTCAATGGTGGTGTACAACAACCAAGAATGCGAAGAAGGC
  V  V  V  K  G  N  V  N  G  G  V  Q  Q  P  R  M  R  R  Q
   190    210    230
AATCCCTTCGCAGGCGCGCTAACAGAGTTCAGCCAGTGGTTATGGTCACGGCCCCTGGGC
  S  L  R  R  R  A  N  R  V  Q  P  V  V  M  V  T  A  P  G  Q
   250    270    290
AACCCAGGCGCCGAAGACGCAGAAGAGGAGGCAATCGCCGCTCAAGAAGAACTGGAGTTC
  P  R  R  R  R  R  R  G  G  N  R  R  S  R  R  T  G  V  P
   310    330    350
CCCGAGGACGAGGCTCAAGCGAGACATTCGTGTTTACAAAGGACAACCTCGTGGGCAACA
  R  G  R  G  S  S  E  T  F  V  F  T  K  D  N  L  V  G  N  T
   370    390    410
CCCAAGGAAGTTTCACCTTCGGGCCGAGTCTATCAGACTGTCCGGCATTCAAGGATGGAA
  Q  G  S  F  T  F  G  P  S  L  S  D  C  P  A  F  K  D  G  I
   430    450    470
TACTCAAGGCCTACCATGAGTATAAGATCACAAGCATCTTACTTCAGTTCGTCAGCGAGG
  L  K  A  Y  H  E  Y  K  I  T  S  I  L  L  Q  F  V  S  E  A
   490    510    530
CCTCTTCCACCTCCTCCGGTTCCATCGCTTATGAGTTGGACCCCCATTGCAAAGTATCAT
  S  S  T  S  S  G  S  I  A  Y  E  L  D  P  H  C  K  V  S  S
   550    570    590
CCCTCCAGTCCTACGTCAACAAGTTCCAAATTACGAAGGGCGGCGCCAAAACTTATCAAG
  L  Q  S  Y  V  N  K  F  Q  I  T  K  G  G  A  K  T  Y  Q  A
   610    630    650
CGCGGATGATAAACGGGGTAGAATGGCACGATTCTTCTGAGGATCAGTGCCGGATACTGT
  R  M  I  N  G  V  E  W  H  D  S  S  E  D  Q  C  R  I  L  W
   670    690    710

```
GGAAGGGAAATGGAAAATCTTCAGATTCCGCAGGATCCTTCAGAGTCACCATCAAGGTAG
 K  G  N  G  K  S  S  Q  S  A  G  S  F  R  V  T  I  K  V  A
        730            750            770
CTTTGCAAAACCCCAAATAGGTAGACTCCGGATCAGAGCCTAGTCCAAGCCCACAACCAA
 L  Q  N  P  K
        790            810            830
CACCCACTCCAACTCCCCAGAAGCACGAGCGATTTATTGCTTACGTTGGCATACCTATGC
        850            870            890
TAACCATTCAGGCCAGGGAGAACGACGACCAAATCATATTGGGTTCCTTAGGGAGCCAAA
        910            930            950
GGATGAAATATATAGAGGACGAGAACCAGAATTATACAAAGTTTAGTTCTGAGTATTACT
        970            990
CTCAATCGAGCATGCAAGCCGTCCCTATGTATTACTTCAATG
```

3. A bacterial plasmid characterised in that it contains the DNA sequence of Claim 1 or Claim 2.

4. A host organism transformed with the bacterial plasmid of Claim 3.

5. Escherichia coli DH5alpha transformed with the bacterial plasmid of Claim 3.

6. A cloning vector containing the DNA sequence of Claim 1 or Claim 2.

7. A method of producing a duplex DNA sequence which is capable of hybridising with a portion of the genome RNA of at least one isolate of PLRV, comprising:

(a) isolating RNA from purified PLRV particles;

(b) synthesising DNA copies of a portion of the RNA by bringing the RNA into contact with a reverse transcriptase in the presence of a primer under conditions of time and temperature sufficient for the production of complementary DNA (cDNA); and

(c) synthesising duplex DNA by bringing complementary cDNA-RNA hybrids into contact with ribonuclease H and a cDNA-RNA polymerase under conditions of time and temperature sufficient for the production of duplex DNA.

8. A DNA probe for use in the detection of PLRV and characterised as comprising a DNA sequence complementary to a portion of the RNA genome of PLRV and capable of hybridisation therewith.

9. A DNA probe for use in the detection of PLRV and characterised as comprising a DNA sequence complementary to a portion of the RNA genome of PLRV and capable of hybridisation therewith.

10. A method of detection of PLRV in samples containing PLRV and which comprises:

a) nick-translation or oligo-labelling of plasmid DNA containing the PLRV-specific DNA sequence in the presence of radioactive or otherwise labelled deoxyribonucleoside triphosphate;

b) applying samples of suspected infected plants or of suspected viruliferous insects to a support to bind the RNA components;

c) treating the support with the labelled DNA under conditions such that the DNA can hybridise to complementary or substantially complementary RNA sequences; and

d) washing the support under conditions such that essentially only hybridised labelled DNA is retained and detecting this bound DNA.

11. A method of producing RNA that is capable of hybridising with a portion of the genome RNA of at least one isolate of PLRV, comprising:

a) isolating the PLRV specific DNA sequence from the plasmid cloning vector;

b) inserting the PLRV specific DNA sequence into a transcription vector;

c) copying the DNA sequence to produce labelled RNA for use in detecting PLRV RNA as in the above-defined aspect of the invention;

d) applying samples of suspected infected plants or of suspected viruliferous insects to a support, for example a sheet of nitrocellulose or other membrane, to bind the RNA components;

e) treating the support with the labelled RNA under conditions such that the RNA can hybridise to complementary or substantially complementary RNA sequences;

f) washing the support under conditions such that essentially only hybridised labelled RNA is retained and detecting this bound RNA.

12. A method of producing PLRV coat protein comprising culturing a host cell under conditions promoting the synthesis of PLRV coat protein wherein said host cell is further characterised as being transformed by an expression vector comprising the DNA sequence of Claim 1 or Claim 2 in an orientation with a promoter sequence such that in the host the said DNA sequence is expressed to produce PLRV coat protein.

_Fig.1_

*Fig.2*

*Fig.3*

Neu eingereicht / Newly
Nouvellement déposé

## FIG.4

```
          10                    30                    50
ATGAGTACGGTCGTGGTTAAAGGAAATGTCAATGGTGGTGTACAACAACCAAGAATGCGA
M  S  T  V  V  V  K  G  N  V  N  G  G  V  Q  Q  P  R  M  R

          70                    90                   110
AGAAGGCAATCCCTTCGCAGGCGCGCTAACAGAGTTCAGCCAGTGGTTATGGTCACGGCC
R  R  Q  S  L  R  R  R  A  N  R  V  Q  P  V  V  M  V  T  A

         130                   150                   170
CCTGGGCAACCCAGGCGCCGAAGACGCAGAAGAGGAGGCAATCGCCGCTCAAGAAGAACT
P  G  Q  P  R  R  R  R  R  R  G  G  N  R  R  S  R  R  T

         190                   210                   230
GGAGTTCCCCGAGGACGAGGCTCAAGCGAGACATTCGTGTTTACAAAGGACAACCTCGTG
G  V  P  R  G  R  G  S  S  E  T  F  V  F  T  K  D  N  L  V

         250                   270                   290
GGCAACACCCAAGGAAGTTTCACCTTCGGGCCGAGTCTATCAGACTGTCCGGCATTCAAG
G  N  T  Q  G  S  F  T  F  G  P  S  L  S  D  C  P  A  F  K

         310                   330                   350
GATGGAATACTCAAGGCCTACCATGAGTATAAGATCACAAGCATCTTACTTCAGTTCGTC
D  G  I  L  K  A  Y  H  E  Y  K  I  T  S  I  L  L  Q  F  V

         370                   390                   410
AGCGAGGCCTCTTCCACCTCCTCCGGTTCCATCGCTTATGAGTTGGACCCCCATTGCAAA
S  E  A  S  S  T  S  S  G  S  I  A  Y  E  L  D  P  H  C  K

         430                   450                   470
GTATCATCCCTCCAGTCCTACGTCAACAAGTTCCAAATTACGAAGGGCGGCGCCAAAACT
V  S  S  L  Q  S  Y  V  N  K  F  Q  I  T  K  G  G  A  K  T

         490                   510                   530
TATCAAGCGCGGATGATAAACGGGGTAGAATGGCACGATTCTTCTGAGGATCAGTGCCGG
Y  Q  A  R  M  I  N  G  V  E  W  H  D  S  S  E  D  Q  C  R

         550                   570                   590
ATACTGTGGAAGGGAAATGGAAAATCTTCAGATTCCGCAGGATCCTTCAGAGTCACCATC
I  L  W  K  G  N  G  K  S  S  D  S  A  G  S  F  R  V  T  I

         610                   630                   650
AAGGTAGCTTTGCAAAACCCCAAATAGGTAGACTCCGGATCAGAGCCTAGTCCAAGCCCA
K  V  A  L  Q  N  P  K  *  V  D  S  G  S  E  P  S  P  S  P

         670                   690                   710
CAACCAACACCCACTCCAACTCCCCAGAAGCACGAGCGATTTATTGCTTACGTTGGCATA
Q  P  T  P  T  P  T  P  Q  K  H  E  R  F  I  A  Y  V  G  I

         730                   750                   770
CCTATGCTAACCATTCAGGCCAGGGAGAACGACGACCAAATCATATTGGGTTCCTTAGGG
P  M  L  T  I  Q  A  R  E  N  D  D  Q  I  I  L  G  S  L  G
```

# FIG.4 (Cont'd)

```
         790                 810                 830
AGCCAAAGGATGAAATATATAGAGGACGAGAACCAGAATTATACAAAGTTTAGTTCTGAG
  S  Q  R  M  K  Y  I  E  D  E  N  Q  N  Y  T  K  F  S  S  E

         850                 870                 890
TATTACTCTCAATCGAGCATGCAAGCCGTCCCTATGTATTACTTCAATGTCCCGAAAGGG
  Y  Y  S  Q  S  S  M  Q  A  V  P  M  Y  Y  F  N  V  P  K  G

         910                 930                 950
CAATGGTCAGTCGACATCAGCTGCGAAGGGTATCAACCCACTAGCAGCACCTCGGATCCA
  Q  W  S  V  D  I  S  C  E  G  Y  Q  P  T  S  S  T  S  D  P

         970                 990                1010
AACCGGGGTAGGAGTGACGGGATGATCGCGTATTCAAACGCGGATTCCGATTATTGGAAT
  N  R  G  R  S  D  G  M  I  A  Y  S  N  A  D  S  D  Y  W  N

        1030                1050                1070
GTTGGTGAAGCGGATGGTGTCAAAAATTTCGAAGCTACGCAACGATAACACCTACCGCCAA
  V  G  E  A  D  G  V  K  I  S  K  L  R  N  D  N  T  Y  R  Q

        1090                1110                1130
GGTCACCCAGAACTTGAAATTAACTCGTGTCATTTTCGCGAGGGTCAACTCCTTGAACGG
  G  H  P  E  L  E  I  N  S  C  H  F  R  E  G  Q  L  L  E  R

        1150                1170                1190
GACGCTACAATTAGCTTCCACGTTGAAGCGCCTACTGATGGGCGATTCTTCCTTGTTGGT
  D  A  T  I  S  F  H  V  E  A  P  T  D  G  R  F  F  L  V  G

        1210                1230                1250
CCTGCCATCCAGAAAACCGCAAAGTATAACTATACTATCTCATACGGTGACTGGACGGAC
  P  A  I  Q  K  T  A  K  Y  N  Y  T  I  S  Y  G  D  W  T  D

        1270                1290                1310
CGAGACATGGAACTGGGGCTGATCACCGTGGTGCTTGATGAACATTTAGAAGGCACTGGT
  R  D  M  E  L  G  L  I  T  V  V  L  D  E  H  L  E  G  T  G

        1330                1350                1370
TCGGCTAACAGAGTGCGGCGGCCCCCACGGGAGGGCCACACCTATATGGCCTCGCCGCAC
  S  A  N  R  V  R  R  P  P  R  E  G  H  T  Y  M  A  S  P  H

        1390                1410                1430
GAACCGGAAGGAAAACCGGTTGGAAATAAACCAAGGGACGAAACCCCGATACAAACGCAG
  E  P  E  G  K  P  V  G  N  K  P  R  D  E  T  P  I  Q  T  Q

        1450                1470                1490
GAAAGACAACCTGATCAAACTCCGTCTGACGACGTATCCGATGCTGGTTCGGTAAACAGC
  E  R  Q  P  D  Q  T  P  S  D  D  V  S  D  A  G  S  V  N  S

        1510                1530                1550
GGCGGCCCAACTGAGTCGCTGCGATTGGAGTTCGGGGTAAACTCAGATAGTACCTACGAT
  G  G  P  T  E  S  L  R  L  E  F  G  V  N  S  D  S  T  Y  D
```

# Fig.4 (Cont'd)

```
      1570                  1590                  1610
GCTACAGTCGATGGTACAGACTGGCCCAGAATTCCTCCACCAAGGCACCCACCTGAACCT
 A   T   V   D   G   T   D   W   P   R   I   P   P   P   R   H   P   P   E   P

      1630                  1650                  1670
AGAGTTTCCGGCAATTCAAGAACTGTCACTGACTTTTCTTCGAAAGCCGATCTATTGGAG
 R   V   S   G   N   S   R   T   V   T   D   F   S   S   K   A   D   L   L   E

      1690                  1710                  1730
AATTGGGATGCCGAACACTTCGACCCTGGTTATTCCAAAGAAGATGTCGCTGCTGCTACT
 N   W   D   A   E   H   F   D   P   G   Y   S   K   E   D   V   A   A   A   T

      1750                  1770                  1790
ATTATAGCGCACGGCAGTATTCAAGATGGGCGAAGTATGTTGGAGAAGAGAGAGGAAAAT
 I   I   A   H   G   S   I   Q   D   G   R   S   M   L   E   K   R   E   E   N

      1810                  1830                  1850
GTCAAGAACAAAACCTCCTCCTGGAAGCCCCCGTCACTTAAAGCGGTGAGCCCAGCCATA
 V   K   N   K   T   S   S   W   K   P   P   S   L   K   A   V   S   P   A   I

      1870                  1890                  1910
GCCAAATTGCGCTCGATTCGCAAATCCCAACCCCTTGAGGGAGGGACCCTTAATAAAGAC
 A   K   L   R   S   I   R   K   S   Q   P   L   E   G   G   T   L   N   K   D

      1930                  1950                  1970
GCCACTGATGGTGTCTCATCTATTGGCAGTGGTTCTCTAACAGGTGGCACGCTTAAGAGG
 A   T   D   G   V   S   S   I   G   S   G   S   L   T   G   G   T   L   K   R

      1990                  2010                  2030
AAGGCAACTATTGAAGAACGTTTACTGCAGACCTTAACAACTGAACAAAGGCTGTGGTAC
 K   A   T   I   E   E   R   L   L   Q   T   L   T   T   E   Q   R   L   W   Y

      2050                  2070                  2090
GAGAATTTTAAGAAAACTAACCCTCCAGCTGCTACCCAATGGCTGTTTGAATATCAGCCA
 E   N   F   K   K   T   N   P   P   A   A   T   Q   W   L   F   E   Y   Q   P

      2110                  2130                  2150
CCTCCCCAAGTAGATAGAAACATAGCTGAAAAGCCATTCCAAGGGAGGAAATGA
 P   P   Q   V   D   R   N   I   A   E   K   P   F   Q   G   R   K
```

32K

23K

−RNA  pSCR
103

_Fig.5_

C  T$_1$  T$_2$  T$_3$  T$_4$  T$_5$  T$_6$  T$_7$

3.2 —
2.9 —
2.1 —

→

0.9 —

## FIG.6

## FIG.7

```
          10                    30                    50
ATAAATTCTTAGCGGGATTTGCTTTAGGATTCTCATCCGCAATCCCATTTTCAGTAGC
          70                    90                   110
CGGTTTATATTTTGTTTACCTAAAGATTTCCTCCCACGTGCGATCAATTGTTAATGAGTA
                                                           M  S  T
         130                   150                   170
CGGTCGTGGTTAAAGGAAATGTCAATGGTGGTGTACAACAACCAAGAATGCGAAGAAGGC
     V  V  V  K  G  N  V  N  G  G  V  Q  Q  P  R  M  R  R  Q
         190                   210                   230
AATCCCTTCGCAGGCGCGCTAACAGAGTTCAGCCAGTGGTTATGGTCACGGCCCCTGGGC
     S  L  R  R  R  A  N  R  V  Q  P  V  V  M  V  T  A  P  G  Q
         250                   270                   290
AACCCAGGCGCCGAAGACGCAGAAGAGGAGGCAATCGCCGCTCAAGAAGAACTGGAGTTC
     P  R  R  R  R  R  R  R  G  G  N  R  R  S  R  R  T  G  V  P
         310                   330                   350
CCCGAGGACGAGGCTCAAGCGAGACATTCGTGTTTACAAAGGACAACCTCGTGGGCAACA
     R  G  R  G  S  S  E  T  F  V  F  T  K  D  N  L  V  G  N  T
         370                   390                   410
CCCAAGGAAGTTTCACCTTCGGGCCGAGTCTATCAGACTGTCCGGCATTCAAGGATGGAA
     Q  G  S  F  T  F  G  P  S  L  S  D  C  P  A  F  K  D  G  I
         430                   450                   470
TACTCAAGGCCTACCATGAGTATAAGATCACAAGCATCTTACTTCAGTTCGTCAGCGAGG
     L  K  A  Y  H  E  Y  K  I  T  S  I  L  L  Q  F  V  S  E  A
         490                   510                   530
CCTCTTCCACCTCCTCCGGTTCCATCGCTTATGAGTTGGACCCCCATTGCAAAGTATCAT
     S  S  T  S  S  G  S  I  A  Y  E  L  D  P  H  C  K  V  S  S
         550                   570                   590
CCCTCCAGTCCTACGTCAACAAGTTCCAAATTACGAAGGGCGGCGCCAAAACTTATCAAG
     L  Q  S  Y  V  N  K  F  Q  I  T  K  G  G  A  K  T  Y  Q  A
         610                   630                   650
CGCGGATGATAAACGGGGTAGAATGGCACGATTCTTCTGAGGATCAGTGCCGGATACTGT
     R  M  I  N  G  V  E  W  H  D  S  S  E  D  Q  C  R  I  L  W
         670                   690                   710
GGAAGGGAAATGGAAAATCTTCAGATTCCGCAGGATCCTTCAGAGTCACCATCAAGGTAG
     K  G  N  G  K  S  S  D  S  A  G  S  F  R  V  T  I  K  V  A
         730                   750                   770
CTTTGCAAAACCCCAAATAGGTAGACTCCGGATCAGAGCCTAGTCCAAGCCCACAACCAA
     L  Q  N  P  K
         790                   810                   830
CACCCACTCCAACTCCCCAGAAGCACGAGCGATTTATTGCTTACGTTGGCATACCTATGC
         850                   870                   890
TAACCATTCAGGCCAGGGAGAACGACGACCAAATCATATTGGTTCCTTAGGGAGCCAAA
         910                   930                   950
GGATGAAATATATAGAGGACGAGAACCAGAATTATACAAAGTTTAGTTCTGAGTATTACT
          970                   990
CTCAATCGAGCATGCAAGCCGTCCCTATGTATTACTTCAATG
```